(19) Europäisches Patentamt
European Patent Office
Office européen des brevets

(11) Veröffentlichungsnummer : **0 057 427**
**B1**

(12) **EUROPÄISCHE PATENTSCHRIFT**

(45) Veröffentlichungstag der Patentschrift : **25.07.84**

(51) Int. Cl.³ : **C 07 D311/70**, C 07 D311/72, C 08 K 5/15

(21) Anmeldenummer : **82100573.3**

(22) Anmeldetag : **28.01.82**

(54) **Chromanderivate.**

(30) Priorität : **04.02.81 DE 3103707**

(43) Veröffentlichungstag der Anmeldung : **11.08.82 Patentblatt 82/32**

(45) Bekanntmachung des Hinweises auf die Patenterteilung : **25.07.84 Patentblatt 84/30**

(84) Benannte Vertragsstaaten : **BE CH DE FR GB IT LI NL**

(56) Entgegenhaltungen :
**AT-B- 210 625**
**DE-A- 2 101 480**
**DE-A- 2 364 141**
**DE-A- 3 010 505**
**DE-B- 2 160 103**
**CHEMICAL ABSTRACTS, Vol. 61, No. 5, 4. Februar 1980, Columbus, Ohio, USA NOAL COHEN, ROCCO J. LEPRESTI, GABRIEL SAUCY "A novel total synthesis of (2R, 4'R, 8'R)-alpha-tocopherol (vitamin E). Construction of chiral chromans from an optically active, nonaromatic precursor." page 805, Spalte 2, Zusammenfassung No. 42 158 n**

(73) Patentinhaber : **BASF Aktiengesellschaft**
**Carl-Bosch-Strasse 38**
**D-6700 Ludwigshafen (DE)**

(72) Erfinder : **Horner, Michael, Dr.**
**Im Ritterbueschel 9**
**D-6730 Neustadt (DE)**
Erfinder : **Horn, Dieter, Dr.**
**Schroederstrasse 69**
**D-6900 Heidelberg (DE)**
Erfinder : **Lueddecke, Erik, Dr.**
**Altholzweg 25**
**D-6700 Ludwigshafen (DE)**
Erfinder : **Teege, Gernot, Dr.**
**Alwin-Mittasch-Platz 12**
**D-6700 Ludwigshafen (DE)**

**EP 0 057 427 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Chromanderivate der allgemeinen Formel I

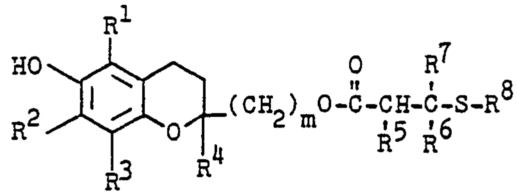

(I)

In dieser Formel bedeuten :
$R^1$-$R^5$ H ; $C_1$-$C_4$-Alkyl
$R^6$, $R^7$ H ; $C_1$-$C_4$-Alkyl ; Aryl
m 1, 2 oder 3
$R^8$ $C_1$-$C_{30}$-Alkyl oder $C_2$-$C_{30}$-Alkenyl

Außerdem betrifft die Erfindung die Herstellung der Verbindungen I, deren Verwendung als Stabilisatoren von organischen Massen, vor allem von Kunststoffen, gegen die Einflüsse von Licht, Wärme und Oxidationsmitteln sowie die mit diesen Stabilisatoren stabilisierten organischen Massen.

Verbindungen mit dem Strukturelement

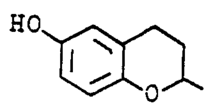

sind als Stabilisatoren für Kunststoffe und organische Massen seit langem bekannt. Der bekannteste Vertreter dieser Substanzklasse, das α-Tocopherol (Vitamin E)

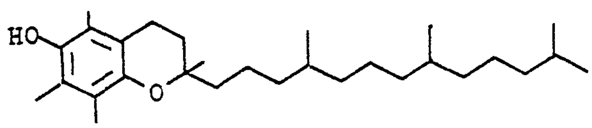

erwies sich als wirkungsvoller Stabilisator für Kunststoffe, wie aus den DE-PS 11 14 319 und 11 36 102 hervorgeht. α-Tocopherol ist jedoch nur relativ schwierig herzustellen und deshalb als Stabilisierungsmittel für die meisten Zwecke zu teuer. Außerdem gibt α-Tocopherol häufig zu unerwünschten Verfärbungen Anlaß. Auch in verarbeitungstechnischer Hinsicht bietet die sehr oxidationsempfindliche ölige Substanz α-Tocopherol Probleme.

Der Erfindung lag daher, wie bei der älteren deutschen Patentanmeldung DE-OS 30 10 505, Die Aufgabe zugrunde, das α-Tocopherol durch im wesentlichen gleich wirkende, aber billigere, Stabilisierungsmittel mit gleichartigen oder besseren Qualitätswerten zu ersetzen.

Demgemäß wurde gefunden, daß sich die eingangs definierten neuen Chromanderivate I hervorragend als Stabilisierungsmittel für organische Massen, darunter vor allem für Kunststoffe, eignen.

Ferner wurden verschiedene Verfahren zur Herstellung der Chromanderivate I gefunden, die im folgenden näher beschrieben werden. Unter den Verbindungen I werden solche bevorzugt, in denen die Reste $R^1$-$R^4$ Methylgruppen bedeuten, da Chromanderivate dieser Struktur besonders leicht zugänglich sind. Verbindungen I mit anderen Resten $R^1$-$R^4$ als jeweils der Methylgruppe sind in analoger Weise zugänglich, und ihre Wirkung als Stabilisatoren ist nach den bisherigen Beobachtungen etwa die gleiche wie bei den Tetramethylchromanderivaten.

Wesentlich für die Stabilisierung ist der Alkyl-bzw. Alkenylrest $R^8$, der definitionsgemäß 1-30 bzw. 2-30, vorzugsweise 7-30 C-Atome enthalten soll, wobei lineare Reste bevorzugt werden.

Die Reste $R^5$-$R^7$ sind vorzugsweise Wasserstoff oder Methylgruppen.

Die Verbindungen I sind durch übliche, sauer katalysierte Veresterung von Alkoholen II

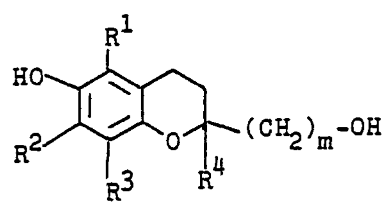

(II)

mit Mercaptopropionsäurederivaten III

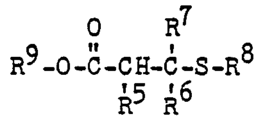

(III)

zugänglich. Die Herstellung der Chromanderivate II ist in der älteren deutschen Patentanmeldung DE-OS 30 10 504 beschrieben, und die Ausgangsverbindungen III sind aus Houben-Weyl, « Methoden der Organischen Chemie », Bd. 9 (1955) S. 124 bekannt oder durch basenkatalysierte Addition von Thiolen VI an die entsprechenden Acrylverbindungen in bekannter Weise erhältlich.

Die Veresterung der Alkohole III ($R^9 = H$) bzw. die Umesterung der Ester III ($R^9 = C_1$-$C_4$-Alkyl) ist in ihren zahlreichen Ausgestaltungen hinsichtlich der als Katalysator dienenden Säure, der Entfernung des Reaktionswassers, der Temperatur und der Reaktionszeit allgemein bekannt, so daß sich nähere Ausführungen hierzu erübrigen.

Eine andere Methode der Herstellung von I besteht in der Addition der Thiole VI

$R^8SH$ (VI)

an die Chromanderivate IV

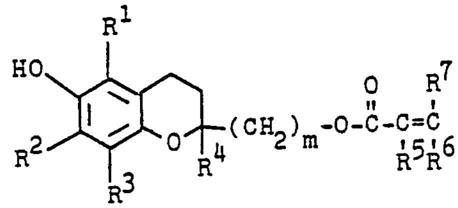

(IV)

Auch diese Reaktion ist an sich bekannt und wird vorzugsweise wie üblich in Gegenwart eines inerten Lösungsmittels sowie von 0,1-10 Gew.%, bezogen auf IV, einer Base vorgenommen.

Als Lösungsmittel eignen sich Toluol, Methylenchlorid, Ethylenchlorid, Tetrahydrofuran, Diethylether und Methyl-tert.-butylether.

Als Basen kommen Natrium- und Kaliumhydroxid in gepulverter Form, Natrium- oder Kaliumalkoholate wie Natriummethylat oder Natriumethylat, Phenolate wie Natriumphenolat, tertiäre Amine wie Triethylamin oder basische Ionenaustauscher in Betracht.

Die Additionsreaktion wird normalerweise bei Raumtemperatur durchgeführt, kann aber auch bei erhöhter Temperatur, etwa bis zu 100 °C, vorgenommen werden, und zwar besonders in den Fällen, in denen die Reste $R^6$ und $R^7$ Alkyl- oder Arylreste darstellen. Die Aufarbeitung erfolgt nach den üblichen Techniken und bedarf deshalb keiner näheren Erläuterung.

Die Verbindungen IV können in üblicher Weise durch Veresterung der Alkohole II mit Acrylsäurederivaten VII

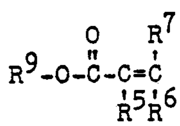

(VII)

hergestellt werden.

Anstelle der Acrylsäurederivate IV kann man auch die entsprechenden β-Halogenpropionsäurederivate V

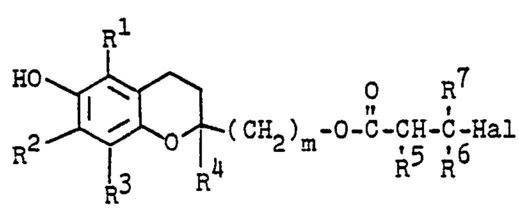

(V)

in denen Hal Chlor oder Brom bedeutet, mit den Thiolen umsetzen, wobei man dann allerdings äquimolare Mengen der Basen verwenden muß. Hinsichtlich der Art der Base und des Lösungsmittels sowie der Reaktionstemperatur und der Aufarbeitung gilt das gleiche wie für die Umsetzung der Acrylsäurederivate.

Die Verbindungen V sind ihrerseits durch Veresterung von II mit den Halogenpropionsäurederivaten

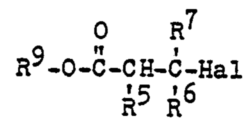

erhältlich.

In allen Fällen kann die Reinigung von I, falls überhaupt erforderlich, bei kristallinen Produkten durch Umkristallisieren, z. B. aus Methanol/Wasser, erfolgen. Im Falle öliger Produkte kann man diese in einem inerten Lösungsmittel lösen und durch Schütteln an ein Adsorptionsmittel wie Kieselgel adsorbieren, wonach man das Adsorbens abfiltriert, das Produkt mit frischem Lösungsmittel desorbiert und das Lösungsmittel wieder abzieht. Als Lösungsmittel eignen sich beispielsweise Diethylether, Methyl-tert.-butylether, Toluol und Methylenchlorid.

Die erfindungsgemäßen Chromanderivate I eignen sich hervorragend als Stabilisatoren von organischen Materialien gegen Schädigungen durch Wärme, Licht und oxidative Einflüsse. Als organische Materialien sind Fette, Öle, Wachse, pharmazeutische und kosmetische Zubereitungen sowie vor allem Kunststoffe zu nennen. Je nach Beanspruchung dieser Materialien verwendet man die Stabilisatoren in Konzentrationen von 0,005 bis 1,0, in der Regel von 0,01 bis 0,5 Gew.%, bezogen auf die Menge des Kunststoffs. Für hochempfindliche Substrate, z. B. Vitamine, können die Konzentrationen bis zu 20 Gew.% ansteigen. Die erfindungsgemäßen Stabilisatoren können allein oder in Mischung mit anderen Stabilisatoren, insbesondere phenolischen Stabilisatoren, wie Neopentylglykol-[3-(3,5-di-tert.-butyl-4-hydroxyphenyl)-propionsäure]-tetraester oder 3-(3,5-Di-tert.-butyl-4-hydroxyphenyl)-propionsäureoctadecylester verwendet werden.

Der besondere Vorzug der erfindungsgemäßen Stabilisatoren liegt darin, daß sie die Verarbeitungs- und Langzeitstabilitätswerte der obengenannten phenolischen Stabilisatoren stark verbessern und somit als Synergisten für diese handelsüblichen Stabilisatoren fungieren. Dies kann dazu genutzt werden, daß die Konzentration der z. T. teuren handelsüblichen Stabilisatoren stark reduziert werden kann, ohne daß die Langzeitstabilisierung darunter leidet. Die Verarbeitungsstabilisierung wird in der Regel sogar deutlich verbessert. Man verwendet die synergistischen Stabilisatoren im allgemeinen in Mengen von 10 bis 500 Gew.%, bezogen auf die Menge der erfindungsgemäßen Chromanderivate I.

Die Chromanderivate I können ferner in Kombination mit anderen Zusätzen wie Calciumstearat verwendet werden, die im allgemeinen in 50 bis 500 Gew.%, bezogen auf die Menge des Derivates I, eingesetzt werden. Derartige Kombinationen ermöglichen sehr niedrige Verfärbungswerte und eine überragende Verarbeitungsstabilisierung in Kunststoffen. Allgemein werden die organischen Materialien durch Stabilisatoren gegen Veränderungen und Zersetzung geschützt, im Falle von Kunststoffen also vornehmlich gegen den Abbau und die unerwünschte Vernetzung der Makromoleküle, Veränderungen, die sich als Alterung, Versprödung, Verfärbung und Herabsetzung der Erweichungstemperatur äußern.

Für die Eignung und Wirksamkeit von Stabilisatoren sind vor allem folgende Kriterien maßgebend :

1. Farbe

Das Substrat soll durch den Stabilisator nicht verfärbt werden. Diese Forderung, die naturgemäß für farblose Kunststoffe von besonderer Wichtigkeit ist, wird durch die erfindungsgemäßen Stabilisatoren bei den meisten Kunststoffen befriedigend bis ausgezeichnet erfüllt, und in aller Regel sind diese Verbindungen herkömmlichen Stabilisierungsmitteln, darunter auch dem α-Tocopherol, überlegen. Die quantitative Ermittlung der Farbeigenschaften kann nach verschiedenen Methoden bestimmt werden, z. B. nach dem Yellowness-Test (ASTMD 1925).

2. Verarbeitungsstabilität

Hierunter ist die Eigenschaftskonstanz von Thermoplasten gegenüber der mechanischen und thermischen Beanspruchung bei Formgebungsverfahren wie der Extrusion und dem Spritzguß zu verstehen. In dieser Beziehung werden mit den erfindungsgemäßen Stabilisatoren besonders gute Ergebnisse erzielt. Eine Maßzahl für die Verarbeitungsstabilität läßt sich aus der Änderung des Schmelzverhaltens des betreffenden Thermoplasten nach mehrmaliger Formgebung unter Wiederaufschmelzen ableiten. Der entsprechende Schmelzindex-Text ist in DIN 53 735 beschrieben. Ein wichtiges Kriterium für die Verarbeitungsstabilität ist auch die Farbkonstanz, die z. B. nach dem Yellowness-Test ermittelt werden kann.

3. Langzeitstabilität

Das Verhalten des Kunststoffes gegen rigorose thermische und oxidative Einflüsse ist ein Indiz für die Dauer der Qualitätskonstanz bei bestimmungsgemäßem Gebrauch, d. h. aus den bei den entsprechenden Tests (DIN 53 383, Blatt 1) ermittelten Werten läßt sich die Lebensdauer des Kunststoffartikels abschätzen. Hier bieten die erfindungsgemäßen Stabilisatoren in Verbindung mit Synergisten Vorteile.

Nähere Angaben zur Qualitätsprüfung der erfindungsgemäßen Stabilisatoren sind den Versuchen über deren Wirkung zu entnehmen.

Beispiele für die Herstellung von Chromanderivaten I und deren Vorprodukte

Im folgenden steht A für den Rest

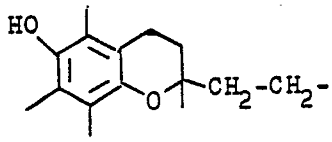

Beispiel 1

Herstellung von A-Acrylat

In einer Destillationsapparatur wurde eine Lösung aus 125 g (0,5 mol) 6-Hydroxy-2,5,7,8-tetramethyl-2-(2-hydroxyethyl)-chroman (A-OH), 258 g (3,0 mol) Acrylsäuremethylester, 2 g Hydrochinon, 8 g p-Toluolsulfonsäure und 125 ml Toluol erhitzt, wobei das Azeotrop aus dem Acrylsäuremethylester und Methanol (Siedebereich 68 °C bis 73 °C) abdestillierte. Nach Ende der Reaktion war das A-OH vollständig umgesetzt, wie sich aus der Dünnschichtchromatographie einer Probe ergab. Nach Abziehen des überschüssigen Acrylsäuremethylesters in einer Drehbandkolonne wurde der verbleibende Rückstand in einer Mischung aus 360 ml Toluol und 40 ml Ethylacetat mit 160 g Kieselgel (70-230 mesh) geschüttelt. Nach Abfiltration und Waschen des Adsorbens mit 300 ml des Lösungsmittelgemisches wurde das Filtrat wieder vom Lösungsmittel befreit. Zurück blieb das A-Acrylat in Form eines gelblichen Öles, das langsam kristallisierte. Ausbeute : 85 %.

Beispiel 2

Herstellung von A-Methacrylat

Nach dem unter Beispiel 1 beschriebenen Verfahren, nur unter Verwendung von 287 g (3,4 mol) Methacrylsäuremethylester anstelle des Acrylesters, fiel das A-Methacrylat als orangefarbenes, langsam kristallisierendes Öl in 91 %iger Ausbeute an.

Beispiel 3

Herstellung von A-p-Methoxyzimtsäureester

Eine Mischung aus 20 g (0,08 mol) A-OH, 14,3 g (0,08 mol) p-Methoxyzimtsäure, 1,5 g p-Toluolsulfonsäure und 800 ml Toluol wurde 20 h unter Rückfluß-Kühlung erhitzt, wobei das Reaktionswasser kontinuierlich entfernt wurde. Nach Abtrennung der p-Toluolsulfonsäure wurde die Mischung zweimal mit je 250 ml gesättigter Natriumhydrogencarbonatlösung und 250 ml Wasser geschüttelt. Anschließend wurde die organische Phase nach dem Trocknen über wassersfreim $Na_2SO_4$ bis auf das Verfahrensprodukt eingeengt. Dieses blieb in Form farbloser Kristalle vom Schmp. 113 bis 114 °C (Methanol) zurück. Die Ausbeute betrug 96 %.

Beispiel 4

A-3,3-Dimethylacrylsäureester

Nach dem unter Beispiel 3 beschriebenen Verfahren, jedoch unter Verwendung von 6,4 g (0,08 mol) 3,3-Dimethylacrylsäure anstelle der p-Methoxyzimtsäure wurde der A-3,3-Dimethylacrylsäureester als bräunliches Öl erhalten.

Beispiele 5 bis 10

Herstellung von β-Mercaptopropionsäurederivaten aus den Acrylsäurederivaten

Zu einer Lösung aus 0,05 mol eines Thiols $R^8SH$, 0,1 g Natriummethylat und 30 ml wasserfreiem Tetrahydrofuran wurde innerhalb von 10 min. eine Lösung von 0,05 mol des A-Acrylsäurederivates in 40 ml wasserfreiem Tetrahydrofuran gegeben, wonach das Gemisch 15 h bei 25° gerührt wurde. Anschließend wurde das Gemisch wie üblich mit Ether extrahiert. Die weitere Aufarbeitung lieferte die Verfahrensprodukte I in den in der Tabelle 1 angegebenen Ausbeuten.

Tabelle 1

Chromanderivate I: 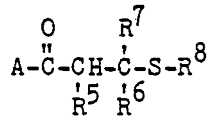

| Bsp. Nr. | 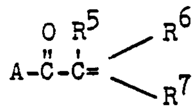 | $R^8$-SH $R^8$ | Chromanderivat I Smp. | Ausbeute |
|---|---|---|---|---|
| 5 | $R^5=R^6=R^7=$ H | -n-Butyl | Öl a) | 80 % |
| 6 | $R^5=R^6=R^7=$ H | n-Octyl | Öl a)b) | 85 % |
| 7 | $R^5=R^6=R^7=$ H | n-Dodecyl | Öl a)b) | 97 % |
| 8 | $R^5=CH_3$; $R^6=R^7=$ H | n-Dodecyl | Öl a)b) | 83 % |
| 9 | $R^5=$H; $R^6=R^7=$ $CH_3$ | n-Dodecyl | Öl b) | 32 % |
| 10 | $R^5=R^6=$ H $R^7$=p-Methoxy-phenyl | n-Dodecyl | Öl b) | 15 % |

a) langsam kristallisierend
b) über Kieselgel gereinigt

Beispiel 11

Herstellung eines Chromanderivates I aus A-OH und III

In einer Destillationsapparatur wurden 25 g (0,1 mol) A-OH und 25,6 g (0,1 mol) 4-Thiapalmitinsäuremethylester in Gegenwart von 0,7 g p-Toluolsulfonsäure und von 300 ml Toluol 3 h unter Rückflußkühlung erhitzt, wobei das anfallende Methanol abdestilliert wurde. Die übliche Aufarbeitung lieferte die Verbindung I mit $R^5 = R^6 = R^7 = H$ und $R^8$ = n-Dodecyl in Form eines langsam kristallisierenden bräunlichen Öles in 85 %iger Ausbeute.

In analoger Weise wurde diese Verbindung aus A-OH und der freien 4-Thiapalmitinsäure hergestellt. In diesem Fall betrug die Ausbeute 80 %.

Versuche zur stabilisierenden Wirkung der Chromanderivate I

1. Farbe von Polypropylen

Hier wurde die Farbqualität als Yellowness-Index YI nach dem Yellowness-Test (ASTMD 1925) gemessen.

Als Testmaterial diente additivfreies dechloriertes Polypropylen, in welches der Stabilisator auf jeweils gleiche Weise eingearbeitet wurde, und das zu Granulat von 15 mm Schichtdicke geformt wurde. Die angegebenen YI-Werte sind jeweils das Mittel aus zwei Messungen. Je höher diese Werte, umso geringer ist die Farbqualität. Die Ergebnisse sind der Tabelle 2 zu entnehmen. Den Werten entsprechen etwa folgende wahrnehmbare Verfärbungen des Testmaterials:

2 nicht erkennbare Verfärbung
3-5 sehr schwache Verfärbung
5-10 schwache, aber bereits deutlich erkennbare Verfärbung
10-20 merkliche Verfärbung
20 Verfärbung

2. Verarbeitungsstabilität von Polypropylen Die Polypropylenproben (gleiches Material wie beim Farbtest) wurden sechsmal extrudiert und granuliert. Aus den Schmelzindices MFI (zur Bestimmung s. DIN 53 735) nach der ersten und der sechsten Extrusion wurde der Quotient $MFI_6/MFI_1$ gebildet. Je größer dieser Quotient ist, umso geringer ist die Verarbeitungsstabilität. Die Farbmessungen entsprechen denen vom Farbtest. Die Ergebnisse sind der Tabelle 3 zu entnehmen.

3. Ofenalterung

Polypropylenplatten von 1 mm Schichtdicke wurden nach DIN 53 383, Blatt 1, der sogenannten Ofenalterung unterworfen, indem die Platten im Wärmeschrank unter Frischluftzufuhr solange auf 140 °C erhitzt werden, bis sie erkennbar versprödeten. Die visuelle Prüfung wurde alle 24 Stunden vorgenommen, d. h. die Alterung wurde hier in Tagen gemessen. Je geringer die Werte, desto geringer die Langzeitstabilität. Die Werte sind Mittelwerte aus zehn Messungen und verstehen sich jeweils mit einer Abweichung mit bis zu etwa 5 %. Die Ergebnisse sind in Tabelle 4 zusammengestellt.

Tabelle 2

Yellowness-Index YI von Polypropylengranulat

| Vers. Nr. | Stabilisator | gemäß Bsp. | Menge Gew.% | YI-Index |
|---|---|---|---|---|
| zum Vergleich | | | | |
| 1 | ohne Stabilisator | - | 0,1 | 1 |
| 2 | Q[x)] | - | 0,1 | 8 |
| 3 | α-Tocopherol | - | 0,1 | 18 |
| 4 | Q | - | 0,1 | 4 |
| | Calciumstearat | | 0,2 | |
| 5 | α-Tocophernol | - | 0,1 | 15 |
| | Calciumstearat | | 0,2 | |
| | DSDP[xx)] | | 0,2 | |
| 6 | α-Tocopherol | - | 0,1 | 16 |
| | Q | | 0,05 | |
| erfindungsgemäß | | | | |
| | $A-O-CO-CH_2-CH_2-S-R^8$ | | | |
| 7 | $R^8$=n-Butyl | 5 | 0,1 | 16 |
| 8 | $R^8$=n-Octyl | 6 | 0,1 | 12 |
| 9 | $R^8$=n-Dodecyl | 7 | 0,1 | 10 |
| 10 | $R^8$=n-Dodecyl | 7 | 0,05 | 10 |
| | Q | | 0,05 | |

x) Pentaerythrityl-tetrakis-3-(3,5-d-tert.-butyl-4-hydroxyphenyl)-propionat
xx) Distearylthiodipropionat

(Siehe Tabelle 3, Seite 8 f.)

Tabelle 3

Schmelzindex-Quotient $MFI_6/MFI_1$ und Yellowness-Index von Polypropylen

| Vers. Nr. | Stabilisator | gemäß Bsp. | Menge Gew.% | $MFI_6/MFI_1$ | YI ($YI_6-YI_1$) |
|---|---|---|---|---|---|
| | zum Vergleich | | | | |
| 11 | ohne Stabilisator | - | 0,1 | 7,3 | 4 |
| 12 | $Q^{x)}$ | - | 0,1 | 2,5 | 13 |
| 13 | α-Tocopherol | - | 0,1 | 1,9 | 10 |
| 14 | Q | - | 0,1 | 2,9 | - |
| | Calciumstearat | - | 0,2 | | |
| 15 | α-Tocopherol | - | 0,1 | 1,5 | 6 |
| | Calciumstearat | - | 0,2 | | |
| | $DSDP^{xx)}$ | - | 0,2 | | |
| 16 | α-Tocopherol | - | 0,1 | 1,5 | 11 |
| | Q | - | 0,05 | | |
| | erfindungsgemäß | | | | |
| | $A-O-CO-CH_2-CH_2-S-R^8$ | | | | |
| 17 | $R^8$=n-Butyl | 5 | 0,1 | 1,5 | 10 |
| 18 | $R^8$=n-Octyl | 6 | 0,1 | 1,3 | 7 |
| 19 | $R^8$=n-Dodecyl | 7 | 0,1 | 1,3 | 5 |
| 20 | $R^8$=n-Dodecyl | 8 | 0,05 | 1,2 | 5 |
| | Q | | 0,05 | | |

x), xx) vgl. Fußnoten zu Tabelle 2

Tabelle 4

Ofenalterung von Polypropylen bei 140 °C

| Vers. Nr. | Stabilisator | gemäß Bsp. | Menge Gew.% | Alterungszeit (h) |
|---|---|---|---|---|
| | zum Vergleich | | | |
| 1 | ohne Stabilisator | - | - | 24 |
| 2 | $Q^{x)}$ | - | 0,1 | 707 |
| 3 | α-Tocopherol | - | 0,1 | 70 |
| 4 | Q | | 0,1 | 734 |
| | Calciumstearat | - | 0,2 | |
| 5 | α-Tocopherol | - | 0,1 | 329 |
| | Calciumstearat | | 0,2 | |
| | $DSDP^{xx)}$ | - | 0,2 | |

Tabelle 4 (Fortsetzung)

| Vers. Nr. | Stabilisator | gemäß Bsp. | Menge Gew.% | Alterungszeit (h) |
|---|---|---|---|---|
| 6 | α-Tocopherol | | 0,1 | 215 |
| | Q | - | 0,05 | |
| | erfindungsgemäß | | | |
| | $A-O-CO-CH_2-CH_2-S-R^8$ | | | |
| 27 | $R^8$=n-Butyl | 5 | 0,1 | 215 |
| 28 | $R^8$=n-Octyl | 6 | 0,1 | 340 |
| 29 | $R^8$=n-Dodecyl | 7 | 0,1 | 315 |
| 30 | $R^8$=n-Dodecyl | 8 | 0,05 | 655 |
| | Q | | 0,05 | |

x), xx) vgl. Fußnoten zu Tabelle 2

4. Stabilität von β-Carotin

4.1 Photostabilität

Je 10 mg eines Trockenpulvers, welches aus 88,5 Gewichtsprozent Polyvinylpyrrolidon, 10 Gew.% β-Carotin und 1,5 Gew.% eines Stabilisators bestand, wurden in 10 ml luftgesättigtem Wasser gelöst. Der intensiven Gelbfärbung der klaren Lösung entsprach ein Licht-Absorptionsmaximum von 430 nm. Bei dieser Wellenlänge und 1 cm Schichtdicke der Lösung betrug die Extinktion 0,8, die gleich 100 % gesetzt wurde.

Zur Ermittlung der Stabilität des β-Carotins gegen den Einfluß von Licht wurde die Lösung mit monochromatischem UV-Licht einer Quecksilberhochdrucklampe bestrahlt (365 nm ; 1 mW/cm²). Ein Maß für die Zerstörung des β-Carotins durch diese Bestrahlung ist die Entfärbung der Lösung, also die Abnahme der Extinktion. Der Tabelle 5 ist jeweils diejenige Zeit in Minuten zu entnehmen, bei der die Extinktion auf den 1/e-ten Teil, also auf rund 0,4 % abfiel.

4.2 Lagerstabilität

Das β-Carotin-Trockenpulver obiger Zusammensetzung wurde sieben Tage im Dukeln bei 25 °C gelagert. Anschließend wurde die Extinktion in Prozent des ursprünglichen Wertes $E_o$ (= 100 %) bestimmt. Für die Messungen wurden jeweils Lösungen aus 50 mg des Trockenpulvers in 100 ml Chloroform verwendet. Die Ergebnisse dieses Tests gehen ebenfalls aus der Tabelle 5 hervor.

Tabelle 5

Photostabilität

| Vers. Nr. | Stabilisator | gemäß Bsp. | Photostabilität min. | Lagerstabilität % von E |
|---|---|---|---|---|
| zum Vergleich | | | | |
| 1 | ohne Stabilisator | - | 5 | 10 |
| 2 | α-Tocopherol | - | 75 | 31 |
| 3 | Ascorbylpalmitat | - | 22 | 20 |
| 4 | 2,6-Di-tert.butyl-4-methylphenol | - | 80 | 30 |
| erfindungsgemäß | | | | |
| 5 | $A-O-CO-CH_2-CH_2-S$-n-dodecyl | 8 | 92 | 40 |

**Ansprüche**

1. Chromanderivate der allgemeinen Formel I

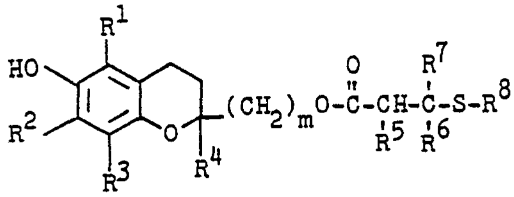

(I)

in der die Substituenten und Indices folgende Bedeutung haben:

$R^1$-$R^5$ H ; $C_1$-$C_4$-Alkyl
$R^6$, $R^7$ H ; $C_1$-$C_4$-Alkyl ; Aryl
m 1, 2 oder 3
$R^8$ $C_1$-$C_{30}$-Alkyl oder $C_2$-$C_{30}$-Alkenyl

2. Chromanderivate gemäß Anspruch 1, wobei die Reste $R^1$-$R^4$ Methylgruppen und die Reste $R^5$, $R^6$ und $R^7$ Wasserstoff bedeuten und wobei m für 2 steht.

3. Verfahren zur Herstellung der Chromanderivate I, dadurch gekennzeichnet, daß man ein Chromanderivat II

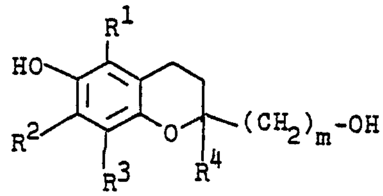

(II)

in an sich bekannter Weise mit einem β-Mercaptopropionsäurederivat der allgemeinen Formel III

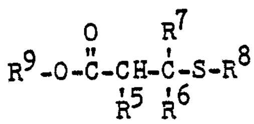

(III)

in der $R^9$ Wasserstoff oder einen $C_1$-$C_4$-Alkylrest bedeutet, umsetzt.

4. Verfahren zur Herstellung der Chromanderivate I, dadurch gekennzeichnet, daß man einen Acrylester IV

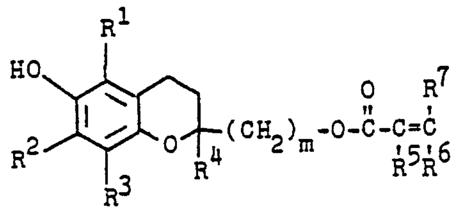

(IV)

oder ein β-Halogenpropionsäurederivat V

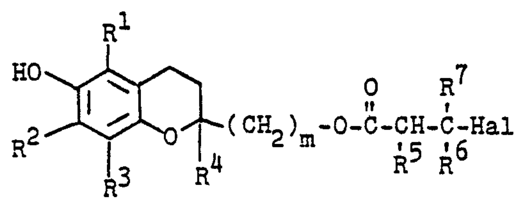

(V)

wobei Hal Chlor oder Brom bedeutet, in an sich bekannter Weise in Gegenwart einer Base sowie eines inerten Lösungsmittels mit einem Thiol VI

$$R^8\text{—}SH \qquad \text{(VI)}$$

umsetzt.

5. Verwendung der Chromanderivate I zum Stabilisieren von organischen Materialien gegen schädigende Einflüsse von Licht, Wärme und Oxidationsmitteln.

6. Verwendung der Chromanderivate I gemäß Anspruch 5 zum Stabilisieren von Kunststoffen in Konzentrationen von 0,01 bis 1,0 Gew.%, bezogen auf die Menge des Kunststoffs.

7. Verwendung der Chromanderivate I gemäß Anspruch 5 zum Stabilisieren von hochempfindlichen organischen Materialien in Konzentrationen von 0,01 bis 20 Gew.%, bezogen auf die Menge dieses Materials.

8. Organische Materialien, gekennzeichnet durch einen Gehalt von 0,01 bis 20 Gew.% eines Chromanderivates I als Stabilisierungsmittel.

9. Organische Materialien gemäß Anspruch 8, gekennzeichnet durch einen Gehalt von 50 bis 500 Gew.%, bezogen auf die Menge des Chromanderivates I, eines synergistisch wirkenden Stabilisierungshilfsmittels.

**Claims**

1. A chroman derivative of the general formula I

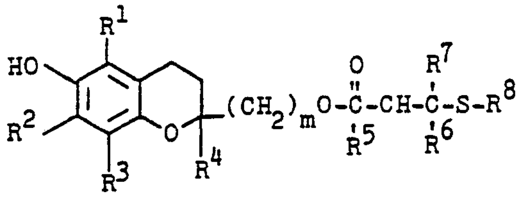

(I)

where
$R^1$, $R^2$, $R^3$, $R^4$ and $R^5$ are each H or $C_1$-$C_4$-alkyl,
$R^6$ and $R^7$ are each H, $C_1$-$C_4$-alkyl or aryl,
m is 1, 2 or 3, and
$R^8$ is $C_1$-$C_{30}$-Alkyl or $C_2$-$C_{30}$-alkenyl.

2. A chroman derivative as claimed in claim 1, where $R^1$, $R^2$, $R^3$ and $R^4$ are each methyl, $R^5$, $R^6$ and $R^7$ are each hydrogen, and m is 2.

3. A process for the preparation of a chroman derivative I, wherein a chroman derivative II

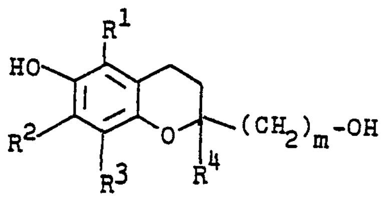

(II)

is reacted in a conventional manner with a β-mercaptopropionic acid derivative of the general formula III

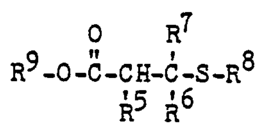

(III)

where $R^9$ is hydrogen or $C_1$-$C_4$-alkyl.

4. A process for the preparation of a chroman derivative I, wherein an acrylate IV

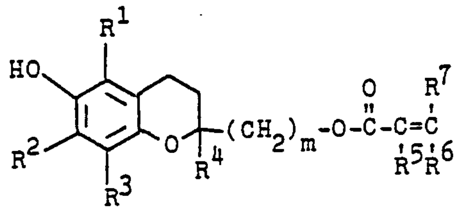

(IV)

or a β-halopropionic acid derivative V

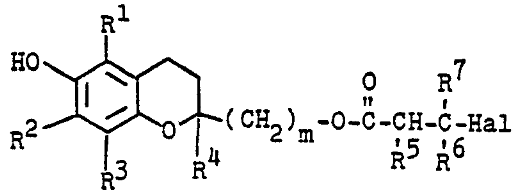

(V)

where Hal is chlorine or bromine, is reacted in a conventional manner, in the presence of a base and of an inert solvent, with a thiol VI

$$R^8\text{—}SH \qquad (VI)$$

5. Use of a chroman derivative I for stabilizing organic materials against the detrimental effects of light, heat and oxidizing agents.

6. Use of a chroman derivative I as claimed in claim 5 for stabilizing plastics, in a concentration of from 0.01 to 1.0 % by weight, based on the plastic.

7. Use of a chroman derivative I as claimed in claim 5 for stabilizing very sensitive organic materials, in a concentration of from 0.01 to 20 % by weight, based on the organic material.

8. An organic material containing from 0.01 to 20 % by weight of a chroman derivative I as stabilizer.

9. An organic material as claimed in claim 8, containing from 50 to 500 % by weight, based on the chroman derivative I, of a synergistic stabilizing auxiliary.

**Revendications**

1. Dérivés du chromane de formule générale I

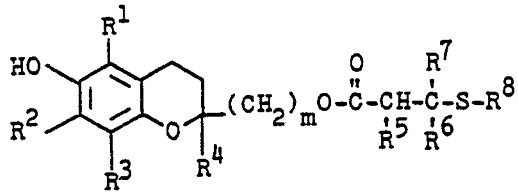

(I)

dans laquelle les substituants et indices ont les significations suivantes :

$R^1$-$R^5$ H ; alcoyle en $C_1$ à $C_4$

$R^6$, $R^7$ H ; alcoyle en $C_1$ à $C_4$ ; aryle

m 1, 2 ou 3

$R^8$ alcoyle en $C_1$ à $C_{30}$ ou alcényle en $C_2$ à $C_{30}$.

2. Dérivés du chromane selon la revendication 1, caractérisés en ce que les radicaux $R^1$ à $R^4$ représentent chacun un groupe méthyle, les radicaux $R^5$, $R^6$ et $R^7$ représentent chacun un atome d'hydrogène et m est mis pour 2.

3. Procédé pour la préparation des dérivés du chromane I, caractérisé en ce qu'on fait réagir un dérivé du chromane II

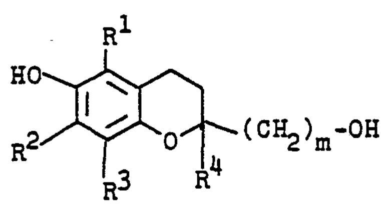

(II)

de façon connue en soi avec un dérivé d'acide β-mercaptopropionique de formule générale III

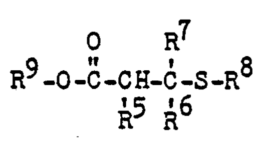

(III)

dans laquelle $R^9$ représente un atome d'hydrogène ou un radical alcoyle en $C_1$ à $C_4$.

4. Procédé pour la préparation des dérivés du chromane I, caractérisé en ce qu'on fait réagir un ester acrylique IV

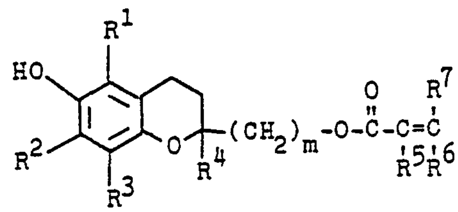

(IV)

ou un dérivé d'acide β-halogénopropionique V

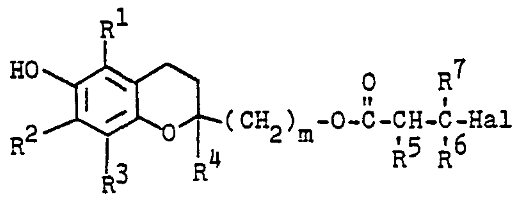

(V)

dans lequel Hal représennte un atome de chlore ou de brome, de façon connue en soi, en présence d'une base ainsi que d'un solvant inerte, avec un thiol VI

$R^8$—SH (VI)

5. Utilisation des dérivés du chromane I pour la stabilisation de matières organiques contre les influences nuisibles de la lumière, de la chaleur et d'agents d'oxydation.

6. Utilisation des dérivés du chromane I selon la revendication 5 pour la stabilisation de matières plastiques, dans des concentrations de 0,01 à 1,0 % en poids, par rapport à la quantité de la matière plastique.

7. Utilisation des dérivés du chromane I selon la revendication 5 pour la stabilisation de matières organiques très sensibles, dans des concentrations de 0,01 à 20 % en poids, par rapport à la quantité de ces matières.

8. Matières organiques, caractérisées par une teneur de 0,01 à 20 % en poids d'un dérivé du chromane I servant d'agent de stabilisation.

9. Matières organiques selon la revendication 8, caractérisées par une teneur de 50 à 500 % en poids, par rapport à la quantité du dérivé du chromane I, d'un agent auxiliaire de stabilisation à action synergique.